(19) Europäisches Patentamt
European Patent Office
Office européen des brevets

(11) EP 1 240 140 B1

(12) EUROPÄISCHE PATENTSCHRIFT

(45) Veröffentlichungstag und Bekanntmachung des Hinweises auf die Patenterteilung:
**17.03.2004 Patentblatt 2004/12**

(21) Anmeldenummer: **00981362.7**

(22) Anmeldetag: **11.12.2000**

(51) Int Cl.[7]: **C07D 207/267**

(86) Internationale Anmeldenummer:
**PCT/EP2000/012515**

(87) Internationale Veröffentlichungsnummer:
**WO 2001/046141 (28.06.2001 Gazette 2001/26)**

(54) **VERFAHREN ZUR HERSTELLUNG VON N-ALKENYL-AMIDEN**

METHOD FOR THE PRODUCTION OF N-ALKENYL AMIDES

PROCEDE DE PRODUCTION DE N-ALCENYLAMIDES

(84) Benannte Vertragsstaaten:
**AT BE CH CY DE DK ES FI FR GB GR IE IT LI LU MC NL PT SE TR**

(30) Priorität: **22.12.1999 DE 19962138**

(43) Veröffentlichungstag der Anmeldung:
**18.09.2002 Patentblatt 2002/38**

(73) Patentinhaber: **BASF AKTIENGESELLSCHAFT**
**67056 Ludwigshafen (DE)**

(72) Erfinder:
- **PREISS, Thomas**
**67256 Weisenheim am Sand (DE)**
- **BÖTTCHER, Arnd**
**67227 Frankenthal (DE)**
- **PINKOS, Rolf**
**67089 Bad Dürkheim (DE)**
- **LORENZ, Rudolf, Erich**
**67063 Ludwigshafen (DE)**

(56) Entgegenhaltungen:
**US-A- 5 665 889** **US-A- 5 670 639**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 240 140 B1

Printed by Jouve, 75001 PARIS (FR)

## Beschreibung

Die vorliegende Erfindung betrifft ein verbessertes Verfahren zur Herstellung von N-Alkenyl-amiden durch Umsetzung der entsprechenden NH-Amide mit Acetylenen in der Flüssigphase in Gegenwart basischer Alkalimetallverbindungen und eines Cokatalysators.

N-Alkenyl-amide werden eingesetzt als Monomere bei der Herstellung von Kunststoffen und Lacken. Polyvinylamide dienen beispielsweise als Waschhilfsmittel, als Hilfsstoffe in kosmetischen und medizinischen Produkten sowie zur Stabilisierung und Klärfiltration von Bieren und Fruchtsäften. Polyvinyl-lactame, insbesondere Polyvinylpyrrolidon-Polymere, besitzen eine breite Anwendung und dienen beispielsweise als Dispergiermittel für Pigmente, als Waschhilfsmittel, als Hilfsstoff in kosmetischen und medizinischen Produkten sowie als Hilfsstoff in der Textilverarbeitung und Klebstofftechnik.

Die technische Herstellung von N-Alkenyl-lactamen erfolgt durch Umsetzung der entsprechenden NH-Lactame mit Acetylenen in Gegenwart basischer Katalysatoren (siehe W. Reppe et al., Justus Liebigs Ann. Chem., 601 (1956) Seite 135-8 und DE-Auslegeschrift 1 163 835).

Die DE-Offenlegungsschrift 3 215 093 offenbart ein Verfahren zur Vinylierung von 2-Pyrrolidon mit Ethin in Gegenwart basischer Katalysatoren, bei dem zusätzlich eine Polyoxyalkylen-Verbindung als Cokatalysator zugegen ist. Als geeignete Polyoxyalkylen-Verbindungen sind genannt Kronenether (z.B. 18-Krone-6), Polyoxyethylen, Polyoxypropylen, wahlweise abgeschlossen durch Alkyl oder Phenylgruppen. Erreicht wurden Umsätze bis 63% und Selektivitäten um 90%, was einer Ausbeute von maximal 57% entspricht. Die Bildung polymerer Rückstände wird vermindert. Bei den genannten Cokatalysatoren handelt es sich jedoch um teuere Einsatzstoffe, welche in der Regel nicht wiedergewonnen werden können, da sie aufgrund ihrer hohen Siedepunkte mit den polymeren Nebenprodukten im Destillationssumpf verbleiben. Zudem sind sie unter den Reaktionsbedingungen im stark basischen Milieu nicht stabil.

Das US-Patent 5,665,889 beschreibt ein Verfahren zur Herstellung von N-Vinyl-2-pyrrolidon aus 2-Pyrrolidon und Ethin in Gegenwart basischer Alkalimetallverbindungen, bei dem als Cokatalysatoren Ether-Oligomere mit Hydroxy-Endgruppen, wie beispielsweise Polytetrahydrofuran, oder lineare Diole mit mindestens 4 Kohlenstoffatomen, wie beispielsweise 1,4-Butandiol, eingesetzt werden. Die Vinylierung erfolgt bei einer Temperatur von 100 bis 200°C, einem Druck von 7,5 bis 30 atm (7,6 bis 30 bar) und einer Reaktionszeit von mehreren Stunden. Beim Einsatz von 1,4-Butandiol wurde selbst nach 4 Stunden Reaktionszeit nur eine Ausbeute von 77,2% erreicht. Erfindungsgemäß wurde erkannt, daß diese Cokatalysatoren aufgrund ihrer hohen Siedepunkte in der Regel nicht von den polymeren Nebenprodukten abgetrennt werden können, beziehungsweise beim Einsatz von 1,4-Butandiol nur durch aufwendige destillative oder chemische Methoden vom Wertprodukt zu trennen sind.

Es bestand daher die Aufgabe, ein Verfahren zur Herstellung von N-Alkenyl-amiden zu entwickeln, welches die aufgeführten Nachteile nicht besitzt, Ausbeuten von größer 80% erlaubt und das Reinprodukt in einfacher Weise zugänglich macht.

Demgemäß wurde ein Verfahren zur Herstellung von N-Alkenyl-amiden durch Umsetzung der entsprechenden NH-Amide mit Acetylenen in der Flüssigphase in Gegenwart basischer Alkalimetallverbindungen und eines Cokatalysators gefunden, das dadurch gekennzeichnet ist, daß man als Cokatalysator Diole der allgemeinen Formel (I)

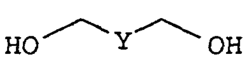 (I),

worin Y
lineares Alkylen $(CH_2)_a$, mit a gleich 0 oder 1; oder
Cycloalkylen mit 3 bis 12 Ring-C-Atomen,
bedeutet, deren Mono-alkenyl-ether, deren Di-alkenyl-ether oder Gemische davon, einsetzt.

Nach dem erfindungsgemäßen Verfahren lassen sich N-Alkenyl-amide aus den entsprechenden NH-Amiden und Acetylenen in Gegenwart basischer Alkalimetallverbindungen und eines preiswerten Cokatalysators, welcher einfach aus dem Reaktionsgemisch wieder abzutrennen ist, in hoher Selektivität und hoher Ausbeute gewinnen.

Die beim erfindungsgemäßen Verfahren bevorzugt einzusetzenden Cokatalysatoren (I) sind beispielsweise 1,2-Ethandiol (Monoethylenglykol), 1,3-Propandiol, 1,1-Bis(hydroxymethyl)-cyclopropan, 1,2-Bis(hydroxymethyl)-cyclopropan, 1,1-Bis(hydroxymethyl)-cyclobutan, 1,2-Bis(hydroxymethyl)-cyclobutan, 1,3-Bis(hydroxymethyl)-cyclobutan, 1,1-Bis(hydroxymethyl)-cyclopentan, 1,2-Bis(hydroxymethyl)-cyclopentan, 1,3-Bis(hydroxymethyl)-cyclopentan, 1,1-Bis(hydroxymethyl)-cyclohexan, 1,2-Bis(hydroxymethyl)-cyclohexan, 1,3-Bis(hydroxymethyl)-cyclohexan, 1,4-Bis (hydroxymethyl)-cyclohexan, 1,1-Bis(hydroxymethyl)-cycloheptan, 1,2-Bis(hydroxymethyl)-cycloheptan, 1,3-Bis(hydroxymethyl)-cycloheptan, 1,4-Bis(hydroxymethyl)-cycloheptan, 1,1-Bis(hydroxymethyl)-cyclooctan, 1,2-Bis(hydroxymethyl)-cyclooctan, 1,3-Bis(hydroxymethyl)-cyclooctan, 1,4-Bis(hydroxymethyl)-cyclooctan oder 1,5-Bis(hydroxymethyl)-cyclooctan.

Besonders bevorzugt eingesetzt werden 1,2-Ethandiol (Monoethylenglykol), 1,3-Propandiol und 1,4-Bis(hydroxymethyl)-cyclohexan und/oder dessen Mono-alkenyl-ether und/oder dessen Di-alkenyl-ether oder Gemische davon. Ganz besonders bevorzugt eingesetzt werden 1,2-Ethandiol (Monoethylenglykol), 1,4-Bis(hydroxymethyl)-cyclohexan und/oder dessen Mono-alkenyl-ether und/oder dessen Di-alkenyl-ether oder Gemische davon.

Als bevorzugte Alkenyl-Gruppen der Alkenyl-ether sind unverzweigte und verzweigte Kohlenwasserstoffreste mit 2 bis 6 C-Atomen und einer Doppelbindung zu nennen. Besonders bevorzugt sind all jene Ether zu nennen, welche sich unter den Reaktionsbedingungen aus den genannten Diolen und den zugesetzten Acetylenen bilden. Ganz besonders bevorzugt sind die Vinyl-ether (Ethenyl-ether) und (1-Methyl-ethenyl)-ether, insbesondere die Vinyl-ether (Ethenyl-ether) zu nennen.

In einer besonders bevorzugten Ausführungsform wird der Cokatalysator in seiner diolischen Form (I) dem Reaktionssystem zugefügt, wobei sich dann unter den Reaktionsbedingungen die entsprechenden Mono- und/oder Di-alkenyl-ether bilden können.

Demnach sind als ganz besonders bevorzugte Cokatalysatoren 1,2-Ethandiol und die durch die ganz besonders bevorzugte Umsetzung mit Ethin sich bildenden Vinylether 1-Vinyloxy-2-ethanol, 1,2-Divinyloxy-ethan sowie 1,4-Bis(hydroxymethyl)-cyclohexan und die durch die ganz besonders bevorzugte Umsetzung mit Ethin sich bildenden Vinylether 1-Vinyloxymethyl-4-hydroxymethyl-cyclohexan, 1,4-Bis(vinyloxymethyl)-cyclohexan zu nennen.

Die im erfindungsgemäßen Verfahren zu verwendenden Cokatalysatoren zeichnen sich gegenüber den Cokatalysatoren nach dem Stand der Technik durch ihre leichte Abtrennbarkeit vom Reaktionsgemisch, insbesondere von den N-Alkenyl-amid-Wertprodukten aus. Dabei ist nicht nur auf eine leichte Abtrennbarkeit des eingesetzten Cokatalysators in seiner diolischen Form zu achten, sondern vor allem auch auf eine leichte Abtrennbarkeit von dessen Reaktionsprodukten. Es ist allgemein bekannt, daß Alkohole bei erhöhter Temperatur und erhöhtem Druck in der Flüssigphase in Gegenwart basischer Katalysatoren mit Acetylenen alkenyliert werden können. So setzen sich auch die erfindungsgemäß zu verwendenden diolischen Cokatalysatoren während der NH-Lactam-Alkenylierung mit den Acetylenen um, wobei sie dabei keinesfalls an ihrer positiven Wirkung verlieren. Bei der Alkenylierung der erfindungsgemäß zu verwendenden Cokatalysatoren handelt es sich um eine Konsekutivreaktion, bei der erst die eine OH-Gruppe und anschließend gegebenenfalls die andere OH-Gruppe alkenyliert werden. Das Reaktionsschema ist im folgenden dargestellt, wobei zur Vereinfachung nur der besonders bevorzugte Fall der Alkenylierung mit Ethin dargestellt ist. Die Alkenylierung der Diole mit Acetylenen mit mehr als 2 C-Atomen ist natürlich ebenfalls möglich. X ist wie oben beschrieben definiert.

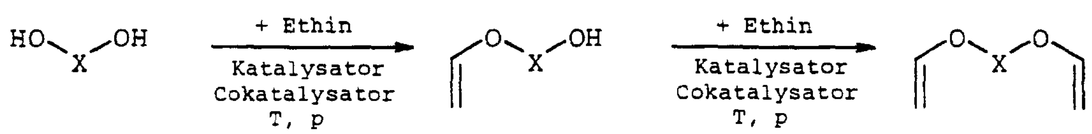


(I) (II) (III)

Da der diolische Cokatalysator (I) in Abhängigkeit von den Reaktionsbedingungen und den eingesetzten Stoffen auch vollständig umgesetzt werden kann, sind somit die Siedepunkte der mono- und di-alkenylierten Verbindungen (II) und (III) in Bezug auf jene der herzustellenden N-Alkenyl-amide von entscheidender Bedeutung. Je weiter diese von denen der N-Alkenyl-amide entfernt liegen, sei es in Richtung niedrigerer oder höherer Siedepunkte, desto einfacher ist die Abtrennung der hergestellten N-Alkenyl-amide.

Als NH-Amide, welche beim erfindungsgemäßen Verfahren als Ausgangsstoff eingesetzt werden, sind sowohl die nicht-cyclischen als auch die cyclischen Amide zu verstehen, welche in ihrer ladungsneutralen Form die Einheit "-CO-NH-" aufweisen.

Aus der Reihe der nicht-cyclischen Amide seien beispielsweise genannt die N-Alkyl-amide der verzweigten und unverzweigten, gesättigten und ungesättigten $C_1$- bis $C_{22}$-Carbonsäuren, mit verzweigten und unverzweigten, gesättigten und ungesättigten $C_1$- bis $C_{10}$-Alkylgruppen am Amid-Stickstoff. Beispiele der nicht-cyclischen NH-Amide sind die Methyl-, Ethyl-, Propyl-, 1-Methylethyl-, Butyl-, 1-Methylpropyl-, 1,1-Dimethylethyl-, Pentyl-, Hexyl, Heptyl-, Octyl-, Nonyl- oder Decyl-amide der Ameisensäure, Essigsäure, Propionsäure, Buttersäure, Valeriansäure, Isovaleriansäure, Pivalinsäure, Capronsäure, 2-Ethylbuttersäure, Önanthsäure, Caprylsäure, 2-Ethylhexansäure, Pelargonsäure, Isononansäure, Caprinsäure, Neodecansäure, Laurinsäure, Myristinsäure, Palmitinsäure, Stearinsäure, Ölsäure, Linolsäure, Linolensäure, Arachidinsäure und Behensäure. Aus der Reihe der nicht-cyclischen NH-Amide bevorzugt

sind N-Methyl-acetamid, N-Methyl-propionamid und N-Ethyl-acetamid.

Besonders bevorzugt ist der Einsatz der cyclischen NH-Amide, welche als NH-Lactame bezeichnet werden. Als NH-Lactame werden beim erfindungsgemäßen Verfahren die 4- bis 12-gliedrigen NH-Lactame, wie beispielsweise 2-Pyrrolidon, 2-Piperidon, ε-Caprolactam und deren Alkylderivate, wie beispielsweise 3-Methyl-2-pyrrolidon, 4-Methyl-2-pyrrolidon, 5-Methyl-2-pyrrolidon, 3-Ethyl-2-pyrrolidon, 3-Propyl-2-pyrrolidon, 3-Butyl-2-pyrrolidon, 3,3-Dimethyl-2-pyrrolidon, 3,5-Dimethyl-2-pyrrolidon, 5,5-Dimethyl-2-pyrrolidon, 3,3,5-Trimethyl-2-pyrrolidon, 5-Methyl-5-ethyl-2-pyrrolidon, 3,4,5-Trimethyl-2-pyrrolidon, 3-Methyl-2-piperidon, 4-Methyl-2-piperidon, 5-Methyl-2-piperidon, 6-Methyl-2-piperidon, 6-Ethyl-2-piperidon, 3,5-Dimethyl-2-piperidon, 4,4-Dimethyl-2-piperidon, 3-Methyl-ε-caprolactam, 4-Methyl-ε-caprolactam, 5-Methyl-ε-caprolactam, 6-Methyl-ε-caprolactam, 7-Methyl-ε-caprolactam, 3-Ethyl-ε-caprolactam, 3-Propyl-ε-caprolactam, 3-Butyl-ε-caprolactam, 3,3-Dimethyl-ε-caprolactam oder 7,7-Dimethyl-ε-caprolactam eingesetzt.

Bevorzugt eingesetzt werden die 4- bis 12-gliedrigen, unsubstituierten NH-Lactame

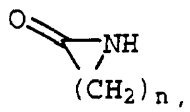

mit n gleich 2 bis 10, wie beispielsweise β-Propiolactam, 2-Pyrrolidon (γ-Butyrolactam), 2-Piperidon (δ-Valerolactam), ε-Caprolactam, sowie deren alkyl-substituierte Derivate. Besonders bevorzugt ist der Einsatz von 2-Pyrrolidon (γ-Butyrolactam), 2-Piperidon (δ-Valerolactam) und ε-Caprolactam.

Als Acetylene werden beim erfindungsgemäßen Verfahren bevorzugt unverzweigte und verzweigte Alkine mit 2 bis 6 C-Atomen und einer endständigen Dreifachbindung eingesetzt, wie beispielsweise Ethin, Propin, 1-Butin, 1-Pentin, 1-Hexin. Besonders bevorzugt ist der Einsatz von Ethin und Propin, insbesondere von Ethin.

Beim ganz besonders bevorzugten 1,2-Ethandiol als Cokatalysator liegt beispielsweise der Siedepunkt der di-vinylierten Verbindung 1,2-Dialkenyloxy-ethan um etwa 88°C niedriger als der von N-Vinyl-2-pyrrolidon, welches im erfindungsgemäßen Verfahren durch die Umsetzung von 2-Pyrrolidon mit Ethin gewonnen werden kann. Auch das mono-vinylierte 1-Vinyloxy-2-ethanol liegt deutlich unterhalb des Siedepunktes von N-Vinyl-2-pyrrolidon. Beim besonders bevorzugten 1,4-Bis(hydroxymethyl)-cyclohexan liegt beispielsweise der Siedepunkt der di-vinylierten Verbindung um etwa 38°C oberhalb des Siedepunktes von N-Vinyl-2-pyrrolidon, so daß auch in diesem Fall eine leichte Abtrennung möglich ist. Sowohl die niedriger siedenden, erfindungsgemäß zu verwendenden Cokatalysatorsysteme als auch die höhersiedenden ermöglichen eine leichte Abtrennbarkeit des N-Alkenyl-lactams.

Der erfindungsgemäß zu verwendende Cokatalysator wird im allgemeinen in einer Menge von 0,1 bis 10 Gew.-%, bezogen auf das eingesetzte NH-Amid, eingesetzt. Besonders bevorzugt ist eine Menge von 0,5 bis 5 Gew.-%.

Der erfindungsgemäß zu verwendende Cokatalysator kann sowohl in seiner diolischen Form (I) als auch in Form seiner Mono-alkenyl-ether als auch in Form seiner Di-alkenyl-ether oder deren Mischungen zugegeben werden.

Als basische Alkalimetallverbindungen, welche auch als Katalysator bezeichnet werden, können beim erfindungsgemäßen Verfahren die Oxide, Hydroxide und/oder Alkoholate von Lithium, Natrium, Kalium, Rubidium und/oder Cäsium sowie deren Gemische eingesetzt werden. Als Alkoholate werden bevorzugt eingesetzt die Verbindungen der niedermolekularen Alkohole, beispielsweise Methanolat, Ethanolat, Propanolat, 1-Methyl-ethanolat, Butanolat, 1-Methyl-propanolat, 2-Methyl-propanolat und 1,1-Dimethyl-ethanolat. Bevorzugt setzt man die Oxide, Hydroxide und/oder Alkoholate von Natrium und/oder Kalium ein. Besonders bevorzugt sind Natriumhydroxid und Kaliumhydroxid. Die basischen Alkalimetallverbindungen können dabei als Feststoffe oder Lösungen in Wasser oder Alkohol eingesetzt werden. Bevorzugt ist der Einsatz fester, wasser- und alkoholfreier Alkalimetallverbindungen. Auch Mischungen verschiedener Alkalimetallverbindungen sind möglich.

Die Umsetzung mit dem Acetylen kann bei einem Molverhältnis zwischen den eingesetzten basischen Alkalimetallverbindungen in Summe zum NH-Amid von 0,02 bis 6,0%, bevorzugt 0,05 bis 4,0%, durchgeführt werden.

Das erfindungsgemäße Verfahren läßt sich wie folgt durchführen:

In einem ersten Schritt des erfindungsgemäßen Verfahrens kann der Cokatalysator im NH-Amid gelöst werden. Die Zugabe kann aber auch an späterer Stelle erfolgen.

Im darauf folgenden Schritt bringt man die basischen Alkalimetallverbindungen in Kontakt mit dem NH-Amid. Es sei darauf hingewiesen, daß das NH-Amid an dieser Stelle bereits die erforderliche Menge an Cokatalysator enthalten kann. Die Zugabe der Alkalimetallverbindung erfolgt beispielsweise durch Lösen im flüssigen NH-Amid oder durch Zugabe einer Lösung der Alkalimetallverbindungen zum NH-Amid erfolgen. Es ist auch möglich, das NH-Amid beziehungsweise die Lösung der Alkalimetallverbindung im NH-Amid durch ein geeignetes Lösungsmittel zu verdünnen, um beispielsweise das Reaktionsverhalten zu beeinflussen. Geeignete Lösungsmittel zeichnen sich dadurch aus, daß sich in ihnen sowohl die NH-Amid als auch die basischen Katalysatoren relativ gut lösen, sie chemisch nicht mit

den eingesetzten Verbindungen reagieren, d.h. vor allem keine aciden Zentren besitzen, welche die basische Gruppen abfangen würden, und sie sich nach der Synthese der N-Alkenyl-amide aus dem System wieder leicht, bevorzugt destillativ, entfernen lassen. Beispiele geeigneter Lösungsmittel sind N-Methylpyrrolidon, Tetrahydrofuran oder Dialkylether von Glykolen, Di-, Oligo- oder Polyglykolen.

Die Herstellung der Lösung der basischen Alkalimetallverbindungen im NH-Amid oder dessen Lösungen erfolgt in der Regel nach den üblichen Verfahren durch Inkontaktbringen des Katalysatorfeststoffs mit der Flüssigkeit unter intensiver Durchmischung. Dadurch wird eine beschleunigte Lösung des Feststoffs erreicht und einer lokalen Erhitzung infolge der Lösungswärme entgegengewirkt. Geeignete Apparate sind dem Fachmann bekannt. Ohne Limitierung seien beispielsweise Rührkessel genannt. Die Flüssigkeit wird vorgelegt und der Katalysatorfeststoff unter intensiver Durchmischung, gegebenenfalls über eine Zeitspanne hinweg, kontinuierlich oder in Portionen zudosiert. Bei der Verwendung von Lösungen der basischen Alkalimetallverbindungen in Wasser oder Alkoholen wird prinzipiell analog verfahren. Auch hier sind dem Fachmann geeignete Methoden bekannt. Es ist auch möglich, den Cokatalysator zusammmen mit der Alkalimetallverbindung zuzugeben.

Die Lösung der basischen Alkalimetallverbindungen im NH-Amid kann sowohl hochkonzentriert als "Katalysator/NH-Amid-Urlösung" oder niedrigkonzentriert als "Katalysator/NH-Amid-Reaktionslösung" hergestellt werden. Bei der hochkonzentrierten "Katalysator/NH-Amid-Urlösung" wird eine hohe Katalysatorkonzentration, welche maximal die Löslichkeitsgrenze erreicht, bei der niedrigkonzentrierten "Katalysator/NH-Amid-Reaktionslösung" die für die Umsetzung mit Acetylen erforderliche Katalysatorkonzentration eingestellt. Natürlich sind auch sämtliche Zwischenstufen möglich.

Bei der Umsetzung der NH-Amide mit den Alkalimetallverbindungen werden in einer Gleichgewichtsreaktion Wasser beziehungsweise Alkohole als flüssige Nebenprodukte gebildet. Wasser beziehungsweise die gebildeten Alkohole verbleiben in Lösung und verhindern aufgrund der Gleichgewichtsbeziehung einen vollständigen Umsatz zwischen dem NH-Amid und den basischen Alkalimetallverbindungen.

Durch eine gezielte Entfernung des gebildeten Reaktionswassers und/oder des gebildeten Reaktionsalkohols wird eine Verschiebung des Gleichgewichts in Richtung des Alkalisalzes des NH-Amids erreicht, so daß die genannte Salzstufe in ausreichender Konzentration erhalten werden kann. Die Zugabe der basischen Alkalimetallverbindungen zum NH-Amid kann sowohl in einem separaten Verfahrensschritt vor als auch während der Entfernung des Reaktionswassers/-alkohols erfolgen. Zudem ist die Zugabe des Cokatalysators in einem separaten Schritt nach Zugabe der Alkalimetallverbindung, vor oder während der Entfernung des Reaktionswassers/-alkohols möglich.

Die vorteilhafte Entfernung des gebildeten Reaktionswassers und/oder des gebildeten Reaktionsalkohols trägt zur Erreichung einer besonders hohen Selektivität und Ausbeute an N-Alkenyl-amiden bei.

Als besonders bevorzugte Methoden zur Entfernung des Reaktionswassers bzw. der niedermolekularen Reaktionsalkohole sind zu nennen die Verdampfung, die Bindung an ein geeignetes Trocknungsmittel (Adsorption) und die Ausschleusung durch eine geeignete Membran. Die genannten Methoden können auch beim Einsatz wässriger oder alkoholhaltiger Katalysatorlösungen angewandt werden.

Bei der Verdampfung macht man sich den großen Unterschied in den Dampfdrücken von Wasser bzw. der niedermolekularen Alkohole und den NH-Amiden zunutze. Bevorzugt erfolgt die Verdampfung des Wassers bzw. Reaktionsalkohols bei erhöhter Temperatur zwischen 50 und 150°C und einem Unterdruck zwischen 0,1 kPa (1 mbar abs) und kleiner Normaldruck. Die Verdampfung kann durch vielfältige Weise erfolgen. Sie kann beispielsweise in einem durchmischten Behälter (z.B. Rührkessel) durch Erwärmung und/oder Anlegen eines Unterdrucks erfolgen. Auch ein Herausstrippen durch Durchleiten eines Inertgases, wie beispielsweise Stickstoff, ist möglich. Die Verdampfung kann auch beim Durchleiten der Lösung durch einen Verdampfer erfolgen. Derartige Geräte sind in der einschlägigen Fachliteratur beschrieben (siehe z.B. Ullmann's Encyclopedia of Industrial Chemistry, 6$^{th}$ edition, 1998 Electronic Release, Chapter "Evaporation"). Als Verdampfungsverfahren besonders bevorzugt ist die Destillation. Sie kann diskontinuierlich, halbkontinuierlich oder kontinuierlich durchgeführt werden. Bei der diskontinuierlichen Destillation werden das NH-Amide, der Katalysator, welcher vollständig oder auch nur partiell gelöst sein kann, und gegebenenfalls der Cokatalysator in der Destillationsblase vorgelegt und durch Erhöhung der Temperatur und/oder Erniedrigung des Druckes das Reaktionswasser bzw. der Reaktionsalkohol abdestilliert. Bei der halbkontinuierlichen Destillation wird beispielsweise eine Lösung des Katalysators im NH-Amid, welches gegebenenfalls den Cokatalysator enthält, dem Kolonnenteil zugeführt und das Reaktionswasser bzw. der Reaktionsalkohol kontinuierlich abdestilliert. Das wasser- bzw. alkoholfreie Produkt sammelt sich dabei in der Destillationsblase an. Die kontinuierlich Destillation unterscheidet sich von der halbkontinuierlichen hauptsächlich dadurch, daß das wasser- bzw. alkoholfreie Produkt kontinuierlich aus dem Sumpf abgeführt wird. Die Destillationen werden bevorzugt bei einem Druck kleiner 0,1 MPa (1 bar abs) durchgeführt.

Beim Einsatz eines Trocknungsmittels nutzt man die exotherme Adsorption kleiner Moleküle an geeigneten hochoberflächigen Feststoffen aus. Besonders hervorzuheben ist hierbei die Entfernung von Wasser. In der Fachliteratur ist eine Vielzahl geeigneter Trocknungsmittel beschrieben (siehe z.B. Ullmann's Encyclopedia of Industrial Chemistry, 6$^{th}$ edition, 1998 Electronic Release, Chapter "Zeolites"). Ohne Limitierung genannt seien zeolithische Molsiebe,

wie beispielsweise vom Typ "13X". Auch die Trocknung kann durch verschiedene Verfahrensweisen erfolgen. In einer Variante befindet sich beispielsweise das Trocknungsmittel direkt im Reaktionssystem, in dem die spätere Umsetzung mit dem Acetylen erfolgt. In einer anderen Variante wird die Lösung durch eine Schüttung des Trocknungsmittels durchgeleitet und erst anschließend in den Alkenylierungsreaktor gegeben.

Bei der dritten genannten Variante, der Ausschleusung über eine Membran, nutzt man den Größenunterschied zwischen Wasser bzw. den niedermolekularen Alkoholen und den tertiären Dialkoholen aus. In einer Ausführungsform befindet sich die Membran direkt im Reaktionssystem, in dem die spätere Umsetzung mit dem Acetylen erfolgt. In einer anderen Ausführungsform wird die Lösung in einem vorgeschaltetem Apparat über eine Membran geleitet. Geeigenete Membrane sind in der einschlägigen Fachliteratur vorbeschrieben (siehe z.B. Ullmann's Encyclopedia of Industrial Chemistry, $6^{th}$ edition, 1998 Electronic Release, Chapter "Membranes and Membran Separation Processes").

Die Entfernung des Reaktionswassers und/oder Reaktionsalkohols erfolgt bevorzugt durch die oben diskutierten Methoden Verdampfung, Adsorption und/oder durch eine Membran. Auch beliebige Kombinationen zwischen den einzelnen Methoden sind möglich und gegebenenfalls sogar vorteilhaft. Ohne Limitierung genannt seien eine zweistufige Destillation, eine Destillation mit nachgeschalteter Adsorption oder eine Entfernung mittels einer Membran mit nachgeschalteter Adsorption. Besonders bevorzugt eingesetzt wird die destillative Entfernung, welche ganz besonders bevorzugt einstufig, bei einem Druck kleiner 0,1 MPa (1 bar abs) durchgeführt wird.

Bei der Entfernung des Reaktionswassers beziehungsweise des Reaktionsalkohols ist es vorteilhaft, wenn ein Restgehalt von kleiner 1 Gew.-%, bevorzugt kleiner 0,5 Gew.-%, besonders bevorzugt kleiner 0,2 Gew.-%, bezogen auf die Gesamtflüssigkeitsmenge, erreicht wird.

Die erfindungsgemäße Zugabe des Cokatalysators kann auch nach der Entfernung des Reaktionswassers bzw. des Reaktionsalkohols erfolgen. Hierbei ist darauf zu achten, daß der zugeführte Cokatalysator frei von Wasser und den niedermolekularen Monoalkoholen, wie beispielsweise Methanol, Ethanol oder Propanol, ist, damit die Wirkung der vorhergehenden Stufe nicht herabgesetzt wird.

Enthält der Cokatalysator Wasser oder niedermolekulare Monoalkohole, so sind diese vor Zugabe abzutrennen. Bevorzugt ist in diesem Falle aber die Zugabe zur NH-Amid/Katalysatorlösung vor der Verfahrensstufe zur Abtrennung des Reaktionswassers bzw. des Reaktionsalkohols.

Die Umsetzung mit dem Acetylen erfolgt durch Inkontaktbringen der oben beschriebenen, NH-Amid-, Katalysator- und Cokatalysator-haltigen, aufbereiteten (d.h. wasser- und mono-alkoholfreien) Lösung mit dem Acetylen in der Flüssigphase. Die sogenannte NH-Amid/Katalysator/Cokatalysator-Lösung kann dabei auch durch ein wasserund mono-alkoholfreies Lösungsmittel verdünnt sein. Als geeignete Lösungsmittel kommen im allgemeinen all jene in Betracht, welche auch bei der Lösung des NH-Amids und der basischen Katalysatoren eingesetzt werden können. Beispiele geeigneter Lösungsmittel sind N-Methylpyrrolidon, Tetrahydrofuran oder Dialkylether von Glykolen, Di-, Oligooder Polyglykolen. Bevorzugt wird die Umsetzung unverdünnt, d.h. ohne Zusatz eines weiteren Lösungsmittels durchgeführt.

Wurde eine Katalysator/NH-Amid-Lösung mit einer Katalysatorkonzentration oberhalb des für die Umsetzung mit Acetylen erforderlichen Gehaltes hergestellt, beispielsweise eine sogenannte "Katalysator/NH-Amid-Urlösung", und erfindungsgemäß behandelt, so ist diese nun mit weiterem, wasser- und alkoholfreien, NH-Amid zu verdünnen. Die Verdünnung kann sowohl außerhalb oder innerhalb des Alkenylierungsreaktors erfolgen. Die erfindungsgemäß behandelte, niedrigkonzentrierte "Katalysator/NH-Amid-Reaktionslösung" kann direkt eingesetzt werden.

Die Umsetzung mit Acetylen kann in verschiedener Art und Weise erfolgen. Beim halbkontinuierlichen Verfahren wird die gesamte NH-Amid/Katalysator/Cokatalysator-Lösung vorgelegt und das Acetylen, entsprechend dem Reaktionsverlauf, zudosiert. Die Produktlösung wird normalerweise erst nach Beendigung der Reaktion entnommen. Beim kontinuierlichen Verfahren werden die NH-Amid/Katalysator/Cokatalysator-Lösung und das Acetylen kontinuierlich zugeführt und die entsprechende Produktlösung kontinuierlich abgeführt.

Die Alkenylierung wird im allgemeinen bei einer Temperatur von 100 bis 200°C, bevorzugt von 130 und 180°C, besonders bevorzugt von 140 bis 160°C durchgeführt. Sie wird im allgemeinen bei einem Acetylendruck von kleiner 5 MPa (50 bar abs), bevorzugt von kleiner 3 MPa (30 bar abs), ganz besonders bevorzugt von kleiner 2,4 MPa (24 bar abs) durchgeführt. Der Gesamtdruck des Systems kann jedoch deutlich höher liegen, da die überstehende Gasatmosphäre beispielsweise noch Inertgase, wie Stickstoff oder Edelgase, welche durch gezieltes Aufpressen eingebracht werden können, enthalten kann. So ist ein Gesamtdruck im System von beispielsweise 20 MPa (200 bar abs) ohne weiteres möglich. Werden höhermolekulare Acetylene eingesetzt, so ist der sich einstellende Acetylendruck sehr niedrig und kann beispielsweise deutlich unterhalb von 0,1 MPa (1 bar abs) liegen. Bei den niedermolekularen Aycetylenen, wie etwa Ethin, Propin und 1-Butin, wird im allgemeinen ein Acetylendruck von größer 0,1 MPa (1 bar abs) eingestellt. Dadurch wird eine wirtschaftliche Raum/Zeit-Ausbeute erreicht. Wird bei der Alkenylierung Ethin als Acetylen eingesetzt, so wird sie bevorzugt bei einem Acetylendruck (Ethindruck) von 0,5 bis 3,0 MPa (5 bis 30 bar abs), besonders bevorzugt von 0,8 bis 2,4 MPa (8 bis 24 bar abs) und ganz besonders bevorzugt von 1,6 bis 2,0 MPa (16 bis 20 bar abs) durchgeführt.

Als Reaktoren für die Alkenylierung können prinzipiell die in der einschlägigen Fachliteratur beschriebenen Apparate für gas-flüssig Umsetzungen eingesetzt werden. Zur Erzielung einer hohen Raum/Zeit-Ausbeute ist eine intensive Durchmischung zwischen der NH-Amid/Katalysator/Cokatalysator-Lösung und dem Acetylen wichtig. Als nicht-einschränkende Beispiele seien genannt Rührkessel, Rührkesselkaskade, Strömungsrohre (bevorzugt mit Einbauten), Blasensäulen und Schlaufenreaktoren. Der Reaktionsaustrag wird nach bekannten Methoden aufgearbeitet. Bevorzugt ist eine Destillation in mehrere Fraktionen. Die Destillationen werden bevorzugt bei einem Druck kleiner 0,1 MPa (1 bar abs) durchgeführt. Besonders bevorzugt werden neben dem N-Alkenyl-amid auch die alkenylierten Cokatalysatoren als Fraktion gewonnen. Je nach Wahl der erfindungsgemäß zu verwendenden Cokatalysatoren werden die alkenylierten Cokatalysatoren in einer niedrigersiedenden oder höhersiedenden Fraktion vor oder nach dem N-Alkenyl-amid abgetrennt. Im folgenden seien, ohne limitierend zu wirken, verschiedene Fraktionen genannt: alkenylierter Cokatalysator (vor oder nach N-Alkenyl-amid), N-Alkenyl-amid, nicht-umgesetztes NH-Amid, diverse Zwischensieder, Leichtsieder und Schwersieder. Je nach Intention können diese als Rohfraktion oder in hoher Reinheit gewonnen werden. Es ist auch möglich, einige Fraktionen zusammenzufassen. Die Destillation kann diskontinuierlich, halbkontinuierlich oder kontinuierlich durchgeführt werden. Zudem kann sie in einer Kolonne, gegebenenfalls mit Seitenabzügen, als auch in mehreren, hintereinandergeschalteten Kolonnen durchgeführt werden. Geeignete Verfahren sind dem Fachmann bekannt. N-Alkenyl-amid kann durch das erfindungsgemäße Verfahren, wie beschrieben, in einfacher Art und Weise mit einem Reinheitsgrad von über 99,8% erhalten werden.

Das gegebenenfalls abgetrennte, nicht-umgesetzte NH-Amid kann beim erfindungsgemäßen Verfahren ohne weitere Reinigungsmaßnahmen rückgeführt werden. Hierzu ist es nicht erforderlich, den Einsatzstoff in hoher Reinheit rückzugewinnen, so daß auch auf eine roh destillierte Fraktion zurückgegriffen werden kann. Es ist jedoch vorteilhaft, die deutlich höher siedenden Produkte abzutrennen.

Beim erfindungsgemäßen Verfahren ist es möglich, die abgetrennten, alkenylierten Cokatalysatoren (Mono- und Di-alkenyl-ether der Cokatalysatoren (I)) rückzuführen. Es ist nicht erforderlich, sie in hoher Reinheit rückzugewinnen, so daß auch auf eine roh destillierte Fraktion zurückgegriffen werden kann. Allerdings ist es vorteilhaft, die deutlich höher siedenden Produkte abzutrennen. Die gegebenenfalls auftretenden Verluste an Cokatalysatoren bzw. deren alkenylierte Verbindungen sind durch Zugabe frischer, bevorzugt diolischer Cokatalysatoren zu ersetzen.

Das erfindungsgemäße Verfahren wird besonders bevorzugt eingesetzt zur Herstellung von

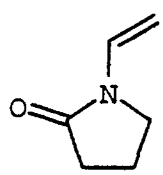

N-Vinyl-2-pyrrolidon
(N-Vinyl-γ-butyrolactam

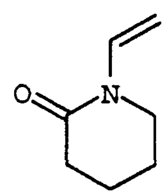

N-Vinyl-2-piperidon
(N-Vinyl-δ-valerolactam)

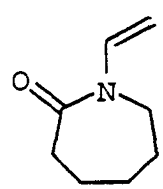

N-Vinyl-ε-caprolactam

sowie deren Mischungen. Ausgangsstoffe hierzu sind die entsprechenden NH-Lactame 2-Pyrrolidon (γ-Butyrolactam), 2-Piperidon (δ-Valerolactam) und ε-Caprolactam. Ganz besonders bevorzugt ist die Herstellung von N-Vinyl-2-pyrollidon.

In einer allgemeinen Ausführungsform wird die basische Alkalimetallverbindung (Katalysator) und der Cokatalysator portionsweise in das flüssige, gegebenenfalls mit Lösungsmittel verdünnte NH-Amid gegeben und durchmischt. Die entstandene Lösung wird sodann über ein zeolithisches Trocknungsmittel geleitet und einem Rührkessel zugeführt. Durch die Gegenwart des Trocknungsmittels wird das Reaktionswasser entfernt. In die nun nahezu wasserfreie Lösung wird bei einer Temperatur von 100 und 200°C unter intensiver Durchmischung das Acetylen eingeleitet. Beim bevorzugten Einsatz von Ethin wird bevorzugt bis zu einem Druck von 2,4 MPa (24 bar abs) eingeleitet. Verbrauchtes Acetylen wird nachgeführt. Nach Beendigung der Acetylenaufnahme wird das Reaktionssystem entspannt. Die Reaktionslösung wird in eine Destillationskolonne überführt und das N-Alkenyl-amid nach Entfernung der leichter siedenden Komponenten über Kopf in hoher Reinheit isoliert.

In einer weiteren allgemeinen Ausführungsform wird in einem Mischbehälter eine nahezu konzentrierte Lösung (d.h. etwa 80% der maximalen Löslichkeit) der basischen Alkalimetallverbindung im NH-Amid hergestellt. Diese Lösung wird kontinuierlich einer Vakuum-Destillationskolonne zugeführt und das gebildete Reaktionswasser über Kopf abgezogen. Die wasserfreie Katalysator/NH-Amid-Lösung wird kontinuierlich aus dem Sumpf entnommen und mit weiterem, wasserfreiem NH-Amid und mit wasserfreiem Cokatalysator versetzt. An dieser Stelle erfolgt auch die Einspeisung der Rückführströme. Die Eduktmischung wird nun einem kontinuierlich arbeitenden Schlaufenreaktor zugeführt. Dort erfolgt die Umsetzung mit dem Acetylen bei einer Temperatur von 100 bis 200°C. Beim bevorzugten Einsatz von Ethin wird bevorzugt bis zu einem Druck von 2,4 MPa (24 bar abs) eingeleitet. Die Reaktionslösung wird kontinuierlich dem Schlaufenreaktor entnommen und destillativ aufgearbeitet. Das N-Alkenyl-amid wird als Reinprodukt isoliert. Zurückgewonnenes, nicht-umgesetztes NH-Amid und der abgetrennte alkenylierte Cokatalysator werden rückgeführt.

In einer dritten, besonders bevorzugten Ausführungsform wird in einem Mischbehälter eine Lösung von etwa 2 Gew.-% Kaliumhydroxid in 2-Pyrrolidon hergestellt und mit etwa 1,0 Gew.-% 1,2-Ethandiol versetzt. Diese Lösung wird kontinuierlich einer Vakuum-Destillationskolonne zugeführt und das gebildete Reaktionswasser über Kopf abgezogen. Die nahezu wasserfreie Lösung wird kontinuierlich aus dem Sumpf entnommen und einem Rührkessel zugeführt. Dort erfolgt die halbkontinuierliche Umsetzung mit dem gasförmigen Ethin bei einer Temperatur von 140 bis 160°C und einem Druck von 1,5 bis 2,0 MPa (15 bis 20 bar abs). Nach Beendigung der Reaktion wird der Reaktorinhalt ausgeschleust und der destillativen Aufarbeitung zugeführt. Es erfolgt eine Auftrennung in Leichtsieder, enthaltend 1-Vinyloxy-2-ethanol und 1,2-Divinyloxy-ethan, N-Vinyl-2-pyrrolidon und Schwersieder. Das N-Vinyl-2-pyrrolidon wird in hoher Reinheit gewonnen.

Das erfindungsgemäße Verfahren ermöglicht die einfache Herstellung von N-Alkenyl-amiden durch Umsetzung der entsprechenden NH-Amide mit Acetylenen in Gegenwart basischer Alkalimetallverbindungen und eines Cokatalysators in sehr hoher Ausbeute und Reinheit. Die herausragenden Vorteile gegenüber dem Stand der Technik sind vor allem:

* Der Einsatz eines, im Falle des besonders bevorzugten 1,2-Ethandiols, sehr preiswerten Cokatalysators.
* Die leichte Abtrennbarkeit des N-Alkenyl-lactams von der Reaktionslösung und die damit erreichbare sehr hohe Reinheit.
* Die Möglichkeit zur Rückgewinnung und zum erneuten Einsatz des Cokatalysators bzw. dessen alkenylierte Verbindungen.

Beispiele

Definitionen

Die in der Beschreibung und den Beispielen angegebenen Werte für Umsatz, Selektivität und Ausbeute sind durch folgende Gleichungen definiert:

$$\text{Umsatz} = [m_{vor}(\text{NH-Amid}) - m_{nach}(\text{NH-Amid})] / m_{vor}(\text{NH-Amid})$$

$$\text{Selektivität} = m_{nach}(\text{N-Alkenyl-amid}) / [m_{vor}(\text{NH-Amid}) - m_{nach}(\text{NH-Amid})]$$

$$\text{Ausbeute} = \text{Umsatz} \times \text{Selektivität} = m_{nach}(\text{N-Alkenyl-amid}) / m_{vor} = (\text{NH-Amid}).$$

Darin bedeuten:

| | |
|---|---|
| $m_{vor}$(NH-Amid) | eingesetzte Masse an NH-Amid |

$m_{nach}$(NH-Amid) nicht-umgesetzte Masse an NH-Amid

$m_{nach}$(N-Alkenyl-amid) Masse an gebildetem N-Alkenyl-amid nach Reindestillation.

Versuchsdurchführung

Die basische Alkalimetallverbindung wurde in das flüssige NH-Amid gegeben und unter Rühren gelöst. Gegebenenfalls wurde noch der Cokatalysator zugegeben. Anschließend wurde das Reaktionswasser bei 0,3 kPa (3 mbar abs) und 100°C entfernt. Der nahezu wasserfreie Reaktionsansatz wurde dann in einen Autoklaven gefüllt und bei Raumtemperatur mit Stickstoff auf 0,2 MPa (2 bar abs) aufgepresst. Nach Erwärmung auf 150°C wurde mit Ethin auf 2,0 MPa (20 bar abs) nachgepresst. Durch Reaktion verbrauchtes Ethin wurde durch kontinuierliches Nachpressen bei 2,0 MPa (20 bar abs) nachgeliefert. Nachdem eine zuvor definierte Menge an Ethin aufgenommen wurde, wurde der Versuch abgebrochen und das Reaktionsprodukt auf destilliert. Die Analyse erfolgte gaschromatografisch.

Die zugegebene Menge an Ethin ist angegeben als relative Molmenge, bezogen auf die Molmenge an eingesetztem NH-Amid.

Beispiele 1 bis 4 (Vergleichsbeispiele ohne Cokatalysator)

Es wurden jeweils 1040 g 2-Pyrrolidon mit 12,48 g KOH versetzt und entsprechend der oben beschriebenen Versuchsdurchführung verfahren.

| Nr. | Cokatalysator | relative Menge an Acetylen | Destillations-Rückstand [Gew.-%] | Umsatz [%] | Selektivität [%] | Ausbeute [%] |
|---|---|---|---|---|---|---|
| 1 | --- | 0,51 | 4,2 | 51,0 | 93,7 | 47,8 |
| 2 | --- | 0,58 | 4,3 | 65,2 | 91,7 | 59,8 |
| 3 | --- | 0,79 | 6,5 | 83,6 | 86,4 | 72,3 |
| 4 | --- | 0,91 | 11,8 | 93,0 | 83,3 | 77,5 |

Mit zunehmender Reaktionszeit steigt zwar der Umsatz an NH-Lactam an, jedoch auch die Bildung von unerwünschten Nebenprodukten. Dies zeigt sich an der Zunahme des höhermolekularen Destillationsrückstands. Der hohe Umsatz an NH-Lactam, welcher über der relativen Menge an zugegebenen Ethin liegt, deutet auf die verstärkte Bildung polymerer Nebenprodukte hin.

Beispiel 5 (Vergleichsbeispiel mit Cokatalysator)

Es wurden 1060 g 2-Pyrrolidon mit 13,46 g KOH und 10,6 g 1,4-Butandiol versetzt und entsprechend der oben beschriebenen Versuchsdurchführung verfahren.

| Nr. | Cokatalysator | relative Menge an Acetylen | Destillations-Rückstand [Gew.-%] | Umsatz [%] | Selektivität [%] | Ausbeute [%] |
|---|---|---|---|---|---|---|
| 5 | 1,4-Butandiol | 0,91 | 7,6 | 89,0 | 91,2 | 81,2 |

Durch destillative Aufarbeitung konnte N-Vinyl-2-pyrrolidon in einer Reinheit von 99,1 Gew.-% erhalten werden.

Beispiel 6 (erfindungsgemäß)

Es wurden 1040 g 2-Pyrrolidon mit 12,48 g KOH und 10,4 g 1,2-Ethandiol versetzt und entsprechend der oben beschriebenen Versuchsdurchführung verfahren.

| Nr. | Cokatalysator | relative Menge an Acetylen | Destillations-Rückstand [Gew.-%] | Umsatz [%] | Selektivität [%] | Ausbeute [%] |
|---|---|---|---|---|---|---|
| 6 | 1,2-Ethandiol | 0,91 | 7,3 | 88,8 | 90,3 | 80,2 |

Durch destillative Aufarbeitung konnte N-Vinyl-2-pyrrolidon in einer Reinheit von > 99,9 Gew.-% erhalten werden.

Beispiel 7 (erfindungsgemäß)

Es wurden 1040 g 2-Pyrrolidon mit 12,48 g KOH und versetzt und 10,4 g 1,4-Bis(hydroxymethyl)-cyclohexan entsprechend der oben beschriebenen Versuchsdurchführung verfahren.

| Nr. | Cokatalysator | relative Menge an Acetylen | Destillations-Rückstand [Gew.-%] | Umsatz [%] | Selektivität [%] | Ausbeute [%] |
|---|---|---|---|---|---|---|
| 7 | 1,4-Bis-(hydroxymethyl)-cyclohexan | 0,91 | 7,3 | 89,5 | 91,5 | 81,9 |

Durch destillative Aufarbeitung konnte N-Vinyl-2-pyrrolidon in einer Reinheit von > 99,9 Gew.-% erhalten werden.

Ein Vergleich der Beispiele 4 bis 7, bei denen die zugegebene relative Menge an Ethin jeweils gleich hoch war, ergibt folgendes Bild:

Ohne Cokatalysator wird ein hoher Destillationsrückstand von 11,8 Gew.-% bei einer Ausbeute von nur 77,5% erhalten. Alle Beispiele mit Cokatalysator zeigen eine deutlich höhere Ausbeute zwischen 80,2 und 81,9%, bei vergleichbar niedrigem Anteil an Destillationsrückstand von 7,3 und 7,6 Gew.-%. Bei Verwendung der erfindungsgemäß zu verwendenden Cokatalysatoren wurde gegenüber denen nach Stand der Technik eine deutlich bessere Abtrennbarkeit des N-Vinyl-2-pyrrolidons erreicht. Dies äußert sich bei gleicher destillativer Aufarbeitung in einer meßbar höheren Reinheit des N-Vinyl-2-pyrrolidons von > 99,9 Gew.-% gegenüber 99,1 Gew.-%.

Somit zeigen die erfindungsgemäß zu verwendenden Cokatalysatoren gegenüber denen der technischen Lehre hinsichtlich Ausbeute und Nebenproduktbildung eine mindestens identische Wirkung, besitzen jedoch in Bezug auf ihre Wirtschaftlichkeit, die verbesserte Abtrennbarkeit von der Reaktionslösung und die erzielbare Reinheit des N-Alkenyl-lactams signifikante Vorteile.

## Patentansprüche

1. Verfahren zur Herstellung von N-Alkenyl-amiden durch Umsetzung der entsprechenden NH-Amide mit Acetylenen in der Flüssigphase in Gegenwart basischer Alkalimetallverbindungen und eines Cokatalysators, **dadurch gekennzeichnet, daß** man als Cokatalysator Diole der allgemeinen Formel (I)

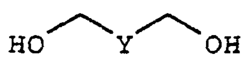 (I),

worin Y
lineares Alkylen $(CH_2)_a$, mit a gleich 0 oder 1; oder
Cycloalkylen mit 3 bis 12 Ring-C-Atomen,
bedeutet, deren Mono-alkenyl-ether, deren Di-alkenyl-ether oder Gemische davon, einsetzt.

2. Verfahren nach Anspruch 1, **dadurch gekennzeichnet, daß** man als Cokatalysator (I) 1,2-Ethandiol, dessen Mono-alkenyl-ether, dessen Di-alkenyl-ether oder Gemische davon, einsetzt.

3. Verfahren nach Anspruch 1, **dadurch gekennzeichnet, daß** man als Cokatalysator (I) 1,4-Bis-(hydroxymethyl)-cyclohexan, dessen Mono-alkenyl-ether, dessen Di-alkenyl-ether oder Gemische davon, einsetzt.

**4.** Verfahren nach den Ansprüchen 1 bis 3, **dadurch gekennzeichnet, daß** man den Cokatalysator (I) in einer Menge von 0,1 bis 10 Gew.-%, bezogen auf das eingesetzte NH-Amid, verwendet.

**5.** Verfahren nach den Ansprüchen 1 bis 4, **dadurch gekennzeichnet, daß** man als basische Alkalimetallverbindungen Natriumhydroxid und/oder Kaliumhydroxid einsetzt.

**6.** Verfahren nach den Ansprüchen 1 bis 5, **dadurch gekennzeichnet, daß** man die basischen Alkalimetallverbindungen in einer Molmenge von 0,05 bis 4,0% der Molmenge des eingesetzten NH-Amids einsetzt.

**7.** Verfahren nach den Ansprüchen 1 bis 6, **dadurch gekennzeichnet, daß** man das bei der Reaktion zwischen den basischen Alkalimetallverbindungen und dem NH-Amid gebildete Reaktionswasser und/oder gebildeten Reaktionsalkohol durch Verdampfung, durch Adsorption und/oder durch eine Membran aus dem System entfernt.

**8.** Verfahren nach den Ansprüchen 1 bis 7, **dadurch gekennzeichnet, daß** man das gebildete Reaktionswasser und/oder den gebildeten Reaktionsalkohol destillativ aus dem System entfernt.

**9.** Verfahren nach den Ansprüchen 1 bis 8, **dadurch gekennzeichnet, daß** man die Umsetzung zwischen den NH-Amiden und den Acetylenen bei einer Temperatur von 100 bis 200°C und einem Acetylendruck von kleiner 5 MPa durchführt.

**10.** Verfahren nach den Ansprüchen 1 bis 9, **dadurch gekennzeichnet, daß** man den Cokatalysator, dessen Mono-alkenyl-ether, dessen Di-alkenyl-ether oder Gemische davon, rückgewinnt und erneut als Cokatalysator einsetzt.

**11.** Verfahren nach den Ansprüchen 1 bis 10, **dadurch gekennzeichnet, daß** man als N-Alkenyl-amide N-Alkenyl-lactame herstellt.

**12.** Verfahren nach Anspruch 11, **dadurch gekennzeichnet, daß** man als N-Alkenyl-lactame N-Vinyl-2-pyrrolidon, N-Vinyl-2-piperidon und/oder N-Vinyl-ε-caprolactam herstellt.

## Claims

**1.** A process for preparing N-alkenyl-amides by reacting the corresponding NH-amides with acetylenes in the liquid phase in the presence of basic alkali metal compounds and of a cocatalyst, which comprises using as the cocatalyst diols of the general formula (I)

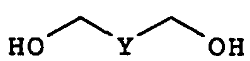 (I),

where Y
is linear alkylene $(CH_2)_a$, where a is 0 or 1; or
cycloalkylene having 3 to 12 ring carbon atoms,
their monoalkenyl ethers, their dialkenyl ethers or mixtures thereof.

**2.** A process as claimed in claim 1, wherein cocatalyst (I) is 1,2-ethanediol, its monoalkenyl ethers, its dialkenyl ethers or mixtures thereof.

**3.** A process as claimed in claim 1, wherein cocatalyst (I) is 1,4-bis-(hydroxymethyl)-cyclohexane, its monoalkenyl ethers, its dialkenyl ethers or mixtures thereof.

**4.** A process as claimed in any of claims 1 to 3, wherein said cocatalyst (I) is used in an amount of from 0.1 to 10% by weight, based on the NH-amide used.

**5.** A process as claimed in any of claims 1 to 4, wherein the basic alkali metal compounds used are sodium hydroxide

and/or potassium hydroxide.

**6.** A process as claimed in any of claims 1 to 5, wherein the basic alkali metal compounds are used in a molar amount of from 0.05 to 4.0% of the molar amount of the NH-amide used.

**7.** A process as claimed in any of claims 1 to 6, wherein the water and/or alcohol of reaction formed in the course of the reaction between the basic alkali metal compounds and the NH-amide is removed from the system by evaporation, by adsorption and/or by a membrane.

**8.** A process as claimed in any of claims 1 to 7, wherein the water and/or alcohol of reaction formed is removed from the system by distillation.

**9.** A process as claimed in any of claims 1 to 8, wherein the reaction between the NH-amides and the acetylenes is carried out at from 100 to 200°C and at an acetylene pressure of less than 5 MPa.

**10.** A process as claimed in any of claims 1 to 9, wherein the cocatalyst, its monoalkenyl ether, its dialkenyl ether or mixture thereof is recovered and reused as cocatalyst.

**11.** A process as claimed in any of claims 1 to 10, wherein the N-alkenyl-amides prepared are N-alkenyl-lactams.

**12.** A process as claimed in claim 11, wherein the N-alkenyl-lactams prepared are N-vinyl-2-pyrrolidone, N-vinyl-2-piperidone and/or N-vinyl-ε-caprolactam.

## Revendications

**1.** Procédé de préparation de N-alcénylamides au moyen de la réaction des amides en NH correspondants avec des acétylènes en phase liquide en présence de composés de métaux alcalins basiques et d'un co-catalyseur, **caractérisé en ce que** l'on utilise, en tant que co-catalyseur, des diols de formule générale (I)

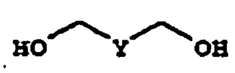

(I),

dans laquelle Y
représente un groupe alkylène linéaire $(CH_2)_a$, a valant 0 ou 1 ; ou un groupe cycloalkylène possédant 3 à 12 atomes de C dans le cycle,
leurs éthers mono-alcényliques, leurs éthers di-alcényliques ou des mélanges de ceux-ci.

**2.** Procédé selon la revendication 1, **caractérisé en ce que** l'on utilise, en tant que co-catalyseur (I), le 1,2-éthanediol, ses éthers mono-alcényliques, ses éthers di-alcényliques ou des mélanges de ceux-ci.

**3.** Procédé selon la revendication 1, **caractérisé en ce que** l'on utilise, en tant que co-catalyseur (I), le 1,4-bis-(hydroxyméthyl)cyclohexane, ses éthers mono-alcényliques, ses éthers di-alcényliques ou des mélanges de ceux-ci.

**4.** Procédé selon les revendications 1 à 3, **caractérisé en ce que** l'on utilise le catalyseur (I) à raison de 0,1% à 10% en poids par rapport à l'amide en NH utilisé.

**5.** Procédé selon les revendications 1 à 4, **caractérisé en ce que** l'on utilise, en tant que composés de métaux alcalins basiques, l'hydroxyde de sodium et/ou l'hydroxyde de potassium.

**6.** Procédé selon les revendications 1 à 5, **caractérisé en ce que** l'on utilise les composés de métaux alcalins basiques à raison de 0,05% à 4,0% de la quantité molaire de l'amide en NH utilisé.

**7.** Procédé selon les revendications 1 à 6, **caractérisé en ce que** l'on élimine du système, lors de la réaction entre les composés de métaux alcalins basiques et l'amide en NH, l'eau réactionnelle et/ou l'alcool réactionnel formés au moyen d'un évaporation, d'une adsorption et/ou à travers une membrane.

8. Procédé selon les revendications 1 à 7, **caractérisé en ce que** l'on élimine l'eau réactionnelle et/ou l'alcool réactionnel formés au moyen d'une distillation.

9. Procédé selon les revendications 1 à 8, **caractérisé en ce que** l'on réalise la réaction entre les amides en NH et les acétylènes à une température allant de 100°C à 200°C et sous une pression d'acétylène inférieure à 5 MPa.

10. Procédé selon les revendications 1 à 9, **caractérisé en ce que** l'on récupère le co-catalyseur, ses éthers mono-alcényliques, ses éthers di-alcényliques ou des mélanges de ceux-ci et **en ce qu'**on les utilise à nouveau en tant que co-catalyseur.

11. Procédé selon les revendications 1 à 10, **caractérisé en ce que** l'on produit, en tant que N-alcényl-amides, la N-alcényl-lactame.

12. Procédé selon la revendication 11, **caractérisé en ce que** l'on produit, en tant que N-alcényl-lactame, la N-vinyl-2-pyrrolidone, la N-vinyl-2-pipéridone et/ou la N-vinyl-ε-caprolactame.